# EUROPEAN PATENT APPLICATION

(11) **EP 4 617 350 A1**
(43) Date of publication of application: **17.09.2025**
(21) Application number: 23888653.5
(22) Date of filing: 06.11.2023
(51) Int. Cl.: C12M 1/00, C12M 1/28, C12N 15/10, C12Q 1/68

(54) **CULTURE VESSEL, MICROORGANISM CULTURE SYSTEM, AND NUCLEIC ACID ANALYSIS SYSTEM**

(30) Priority: 07.11.2022 JP 2022178035
(71) Applicant: Yokogawa Electric Corporation, Tokyo 180-8750 (JP)
(72) Inventor: KUWATA, Masahiro, Musashino-shi, Tokyo 180-8750 (JP)
(74) Representative: Grünecker Patent- und Rechtsanwälte PartG mbB
(86) International application number: PCT/JP2023/039864
(87) International publication number: WO 2024/101305

(57) **Abstract**

A culture vessel (10) includes an opening (11a) that opens in a slit shape, and a vessel body (11) that vertically accommodates a sheet-like object (membrane filter (100)) through the opening (11a).

## Description

### TECHNICAL FIELD

The present invention relates to a culture vessel, a microorganism culture system, and a nucleic acid analysis system.

### BACKGROUND ART

Patent Document 1 below discloses a simple culture vessel as an alternative to traditional petri dishes. This culture vessel has a body and a lid, both of which are formed of a resin sheet, a concave part into which a medium is filled is formed in the body, a convex part that fits into the concave part of the body is formed in the lid, an adhesive layer for sample collection is formed on the surface of the convex part, and when the convex part fits into the concave part, the adhesive layer comes into contact with the medium, and the body and the lid that are connected by a hinge part are produced from the same resin sheet.

### [Citation List]

### [Patent Document]

[Patent Document 1] Japanese Patent No. 5103925

### SUMMARY OF INVENTION

### Technical Problem

Since the vessel body accommodates an object horizontally (flatly), when the medium is a liquid, there is a problem of the medium being likely to spill out of the vessel body, and difficulty in operation. In addition, when the object is in the form of a sheet, at least a part thereof may float on the liquid surface of the medium, and there is a risk of microorganisms attached to the object not being cultured.

The present invention has been made in view of the above problems, and an object of the present invention is to provide a culture vessel which is less likely to spill a liquid, is easy to operate, and can secure submersion of a sheet-like object in a liquid in a vessel body, and a microorganism culture system and a nucleic acid analysis system using the culture vessel.

### Solution to Problem

In order to achieve the above object, a culture vessel according to a first aspect of the present invention has an opening that opens in a slit shape, and a vessel body that vertically accommodates a sheet-like object through the opening.

A culture vessel according to a second aspect of the present invention is the culture vessel according to the first aspect of the present invention, wherein the vessel body has a pair of side wall parts adjacent to each other in a direction in which the opening is shortest, and lower parts of the pair of side wall parts are curved in an arc shape.

A culture vessel according to a third aspect of the present invention is the culture vessel according to the second aspect of the present invention, wherein upper parts of the pair of side wall parts are formed in a rectangular shape that is continuous with the arc shape, and are formed longer in a vertical direction than a vertical dimension of the lower parts.

A culture vessel according to a fourth aspect of the present invention is the culture vessel according to the second or third aspect of the present invention, wherein the distance between facing surfaces of the pair of side wall parts increases from a bottom part of the vessel body toward the opening.

A culture vessel according to a fifth aspect of the present invention is the culture vessel according to any one of the first aspect to the fourth aspect of the present invention, wherein an inclined spout is formed in at least a part of the opening.

A culture vessel according to a sixth aspect of the present invention is the culture vessel according to any one of the first aspect to the fifth aspect of the present invention, including a lid part that covers at least a part of the opening.

A culture vessel according to a seventh aspect of the present invention is the culture vessel according to the sixth aspect of the present invention, wherein the lid part has air permeability.

A culture vessel according to an eighth aspect of the present invention is the culture vessel according to the sixth aspect or the seventh aspect of the present invention, including a sealing part that seals a gap between the lid part and the vessel body.

A culture vessel according to a ninth aspect of the present invention is the culture vessel according to the eighth aspect of the present invention, wherein the lid part is a film, and the sealing part is an adhesive that allows the film to be attached and detached.

A culture vessel according to a tenth aspect of the present invention is the culture vessel according to any one of the first aspect to the ninth aspect of the present invention, wherein a plurality of vessel bodies are connected to each other via connecting parts.

A culture vessel according to an eleventh aspect of the present invention is the culture vessel according to the tenth aspect of the present invention, wherein the connecting part has a breakable weakened part.

A culture vessel according to a twelfth aspect of the present invention is the culture vessel according to any one of the second aspect to the fourth aspect of the present invention, wherein, in the pair of side wall parts, inverted cone-shaped bulging parts that partially widen the opening in the direction in which it is shortest are formed.

A culture vessel according to a thirteenth aspect of the present invention is the culture vessel according to any one of the second aspect to the fourth aspect, and the twelfth aspect of the present invention, wherein, in the pair of side wall parts, protruding parts that protrude toward the inside of the vessel body are formed.

A microorganism culture system according to a fourteenth aspect of the present invention cultures microorganisms using the culture vessel according to any one of the first aspect to the thirteenth aspect of the present invention.

A nucleic acid analysis system according to a fifteenth aspect of the present invention includes the microorganism culture system according to the fourteenth aspect of the present invention, and is configured to analyze nucleic acids extracted from the microorganisms.

### Advantageous Effects of Invention

According to the above aspects of the present invention, it is possible to provide a culture vessel which is less likely to spill a liquid, is easy to operate, and can secure submersion of a sheet-like object in a liquid in a vessel body, and a microorganism culture system and a nucleic acid analysis system using the culture vessel.

### BRIEF DESCRIPTION OF DRAWINGS

[FIG. 1] A schematic view of a nucleic acid analysis system according to a first embodiment.
[FIG. 2] A side view of a culture vessel according to the first embodiment.
[FIG. 3]A cross-sectional view taken along the arrow III-III shown in FIG. 2.
[FIG. 4] A plan view of the culture vessel according to the first embodiment.
[FIG. 5] A partial cut-away side view of a culture vessel according to a second embodiment.
[FIG. 6] A cross-sectional view taken along the arrow VI-VI shown in FIG. 5.
[FIG. 7] A plan view of the culture vessel according to the second embodiment.
[FIG. 8] A plan view of a culture vessel according to a third embodiment.
[FIG. 9] A cross-sectional view taken along the arrow IX-IX shown in FIG. 8.
[FIG. 10] A partial cut-away side view of the culture vessel according to the third embodiment.
[FIG. 11] A side view of a culture vessel according to a fourth embodiment.
[FIG. 12] A cross-sectional view taken along the arrow XII-XII shown in FIG. 11.
[FIG. 13] An exploded perspective view of a culture vessel according to a fifth embodiment.
[FIG. 14] A side view of a vessel body according to the fifth embodiment.
[FIG. 15] A cross-sectional view taken along the arrow XV-XV shown in FIG. 14.
[FIG. 16] A plan view of the vessel body according to the fifth embodiment.

### DESCRIPTION OF EMBODIMENTS

Hereinafter, a culture vessel, a microorganism culture system, and a nucleic acid analysis system according to embodiments of the present invention will be described in detail with reference to the drawings. In the following, first, an overview of the embodiments of the present invention will be described, and subsequently, the embodiments of the present invention will be described in detail.

### [Overview]

Microorganisms such as bacteria may adversely influence the human body, and a method for testing microorganism contamination is desired. Traditionally, in such testing, microorganisms are cultured on agar media in petri dishes, and the number of colonies formed is counted to check contamination and the number of microorganisms. For example, Patent Document 1 discloses a technique relating to a culture vessel that can easily perform sample collection and culture, instead of such traditionally used petri dishes.

On the other hand, in tests using colonies formed by culture, since a culture time from several days to several weeks may be required for colonies to grow to a testable size, a method for testing microorganism contamination more quickly is desired, and various rapid measurement methods have been developed. For example, a technique for extracting nucleic acids from microorganisms by treating cells such as microorganisms at a high temperature and a high pressure and a technique for measuring the sequence of specific nucleic acids by fluorescence through hybridization have been developed.

One such rapid measurement method is a technique for collecting microorganisms through a membrane filter and extracting nucleic acids from a liquid containing the collected microorganisms. However, extraction of nucleic acids from microorganisms collected on the membrane filter has a problem that it is not possible to extract a sufficient amount of nucleic acids for measurement, and it is not possible to detect microorganisms. Therefore, there is a demand for a technique for increasing the amount of microorganisms collected through the membrane filter by culture, extracting nucleic acids from a liquid containing the increased number of microorganisms, and performing testing.

Here, the culture vessel disclosed in Patent Document 1 relates to culture using a solid medium such as an agar medium. Therefore, it is not possible to culture microorganisms collected through the membrane filter in a liquid using the culture vessel and it is not possible to extract nucleic acids from a liquid containing microorganisms and perform testing as described above. In addition, when microorganisms collected through the membrane filter are cultured in a liquid using a petri dish traditionally used for culture, the opening of the petri dish is large, and when an operation such as moving the petri dish is performed, there is a problem of the liquid used for culture being likely to spill, and difficulty in operation.

In addition, when microorganisms are cultured in a liquid, in order to secure a uniform culture state of the microorganisms, it is desirable to secure complete submersion of the membrane filter through which the microorganisms are collected in the liquid. However, when microorganisms collected through the membrane filter are cultured in a liquid using a petri dish traditionally used for culture, at least a part of the membrane filter floats in the petri dish, and it is difficult to secure complete submersion of the membrane filter in the culture solution.

On the other hand, in order to secure complete submersion of the membrane filter in the culture solution, it is necessary to increase the amount of the culture solution put into the petri dish. However, when the amount of the culture solution is increased, since increase in the amount of microorganisms by culture is mainly determined by the culture time, the amount of microorganisms contained in the culture solution remains unchanged after the same culture time has elapsed, and thus the concentration of microorganisms contained in the culture solution decreases. That is, when the amount of the culture solution is increased, since the concentration of microorganisms contained in the liquid is lower for the same culture time, there are cases where the amount of microorganisms reaches a concentration at which microorganisms cannot be detected in the above test, which causes problems in the microorganism test. On the other hand, when culture is performed until the concentration of microorganisms is detectable, there is a problem of the culture time becoming longer.

In order to address such a problem, a culture vessel, a microorganism culture system, and a nucleic acid analysis system according to one embodiment of the present invention have an opening that opens in a slit shape, and a vessel body that vertically accommodates a sheet-like object through the opening. Therefore, the accommodation space can be made deeper without increasing the internal volume of the vessel body, and thus the liquid is less likely to spill, and the operation becomes easier. In addition, when the vessel body vertically accommodates a sheet-like object, the object is less likely to float on the liquid surface. Therefore, it is possible to secure submersion of the object in the liquid even with a small amount of the liquid. Therefore, culture can be efficiently performed in a liquid medium using a sheet-like object.

### [First embodiment]

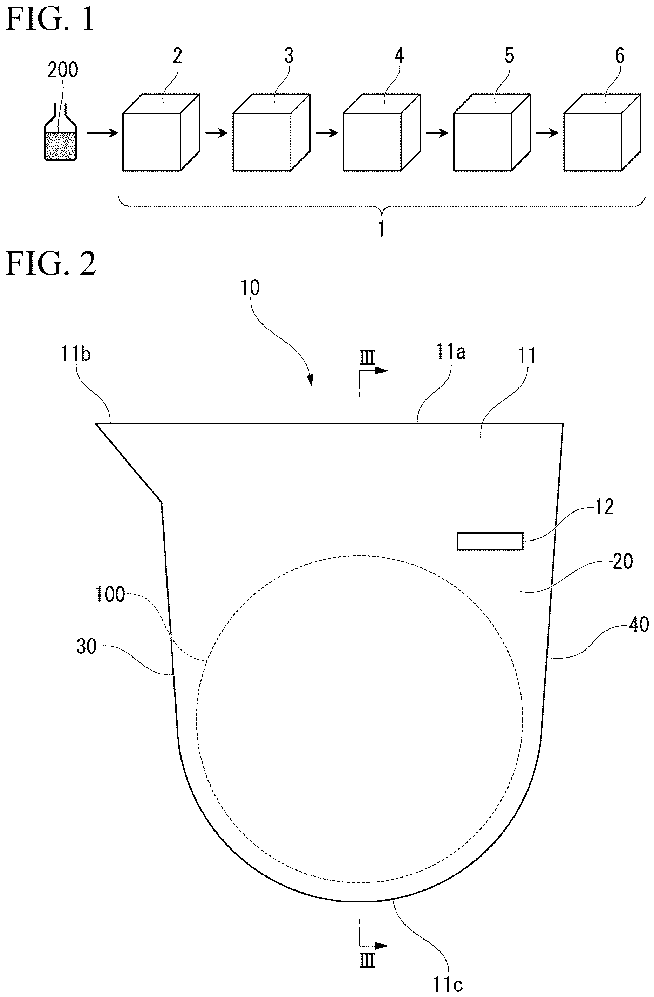

FIG. 1 is a schematic view of a nucleic acid analysis system 1 according to a first embodiment.

As shown in FIG. 1, the nucleic acid analysis system 1 includes a microorganism collection system 2, a microorganism culture system 3, a nucleic acid extraction system 4, a hybridization reaction system 5, and a detection system 6.

The microorganism collection system 2 is a system for collecting, from a sample 200, microorganisms (such as viruses, bacteria, and fungi) contained in the sample 200. For example, if the test is for a beverage, the sample 200 is a produced beverage, water used to produce the beverage, or a liquid used in the process of producing the beverage. Alternatively, the sample 200 may be a liquid from which microorganisms have been collected, such as from a swab used to wipe the test environment in order to check the presence of contamination by microorganisms in the production environment and the degree of contamination.

The microorganisms can be collected by, for example, pressurizing or depressurizing the collected liquid, and filtering it through a membrane filter 100 (sheet-like object) to be described below. For example, when microorganisms are collected, the membrane filter 100 may have a pore size of 0.22 µm to 0.45 µm. After the microorganisms are collected through the membrane filter 100, the membrane filter 100 is accommodated in a culture vessel 10 to be described below, and immersed in a culture solution in which the microorganisms are cultured, and the microorganisms are cultured in the microorganism culture system 3.

The microorganism culture system 3 is a system for culturing microorganisms using the culture vessel 10 to be described below. Examples of microorganism cultures include static culture in which the culture vessel 10 is left stationary for culture and shaking culture in which culture is performed while shaking the culture vessel 10. The culture solution in which the microorganisms are cultured is transferred to the next process (the nucleic acid extraction system 4). The liquid containing the membrane filter 100 may be vibrated, and the liquid in which the microorganisms are suspended may be transferred to the next process.

The nucleic acid extraction system 4 is a system that breaks down (dissolves) the membrane structure of cells in a liquid and extracts nucleic acids from microorganism cells. Here, the sample 200 from which nucleic acids are extracted may be mixed with a liquid containing other nucleic acids that react with the extracted nucleic acids. In addition, the other nucleic acids may be nucleic acids to which a moiety exhibiting a fluorescence, luminescence or quenching effect under specific conditions is imparted in order to detect them in a detection process (the detection system 6) to be described below. These may be mixed with the sample 200 before it is treated in the nucleic acid extraction system 4, or may be mixed with the sample 200 after it is treated in the nucleic acid extraction system 4.

The hybridization reaction system 5 is a system that causes a hybridization reaction in nucleic acids in the sample 200. Here, in this process, for example, the sample 200 is heated to 60°C and stirred to cause a hybridization reaction for matching with the other nucleic acids described above. In this reaction, for example, when the moiety exhibiting a fluorescence, luminescence or quenching effect under specific conditions imparted to the other nucleic acids described above reacts with nucleic acids in the sample 200, the fluorescence, luminescence or quenching effect is exhibited.

Here, when the structure of the other nucleic acids described above is designed to react with specific nucleic acids, for example, it can react only with nucleic acids of specific microorganisms in the sample 200. That is, in the treatment of the hybridization reaction system 5, when other nucleic acids that react with the specific nucleic acids are used, the fluorescence, luminescence or quenching effect imparted to other nucleic acids can be exhibited only when specific microorganisms are contained in the sample 200.

The detection system 6 detects the presence and the degree of the fluorescence, luminescence or quenching effect exhibited in the sample 200 treated in the hybridization reaction system 5. For example, the detection system 6 excites the fluorescence effect exhibited in the nucleic acids in the sample 200 with an excitation laser light, and detects the excited fluorescence with a highly sensitive camera.

Alternatively, the detection system 6 detects the luminescence effect exhibited in the nucleic acids in the sample 200 with a highly sensitive camera. Alternatively, the detection system 6 detects the quenching effect exhibited in the nucleic acids in the sample 200 according to the degree of the fluorescence or luminescence applied to the vicinity of the moiety to which the quenching effect is imparted is quenched with a highly sensitive camera. Regarding this detection method, for example, a method described in Japanese Unexamined Patent Application, First Publication No. 2020-74726 may be used.

The nucleic acid analysis system 1 uses a series of systems as described above to analyze whether specific microorganisms (such as viruses, bacteria, and fungi) are contained in the sample 200 or its concentration.

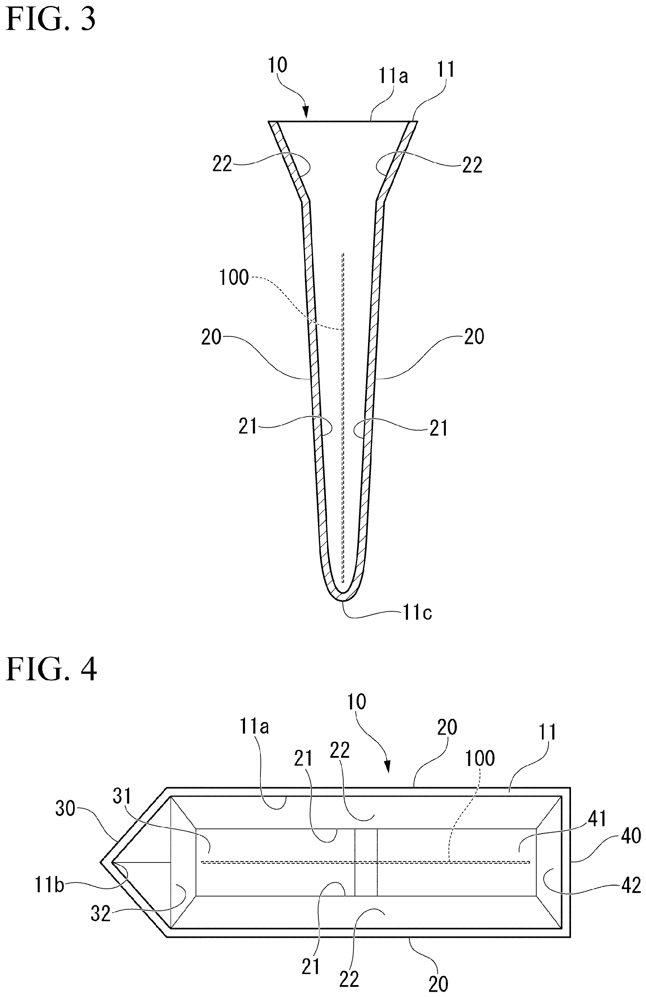

FIG. 2 is a side view of the culture vessel 10 according to the first embodiment. FIG. 3 is a cross-sectional view taken along the arrow III-III shown in FIG. 2. FIG. 4 is a plan view of the culture vessel 10 according to the first embodiment. As shown in these drawings, the culture vessel 10 has an opening 11a that opens in a slit shape, and a vessel body 11 that vertically accommodates a circular membrane filter 100 through the opening 11a.

Here, "slit shape" includes not only a rectangular shape but also an approximately rectangular shape. Approximately rectangular shapes include those in which a part of a rectangular shape (for example, a corner) has an arc shape, a tapered shape, or the like, and also those in which, even if an unevenness is partially formed, the entire surface is considered as a silt. Hereinafter, similarly, "shape" is used in this same broad sense.

As shown in FIG. 2, an inclined spout 11b is formed at one end of the opening 11a in the longitudinal direction. Hereinafter, in the culture vessel 10, the front, rear, left, right, up and down are defined with the side on which the spout 11b is formed as "front," and the side opposite to the spout 11b as "rear." Here, the spout 11b may be formed at both ends of the opening 11a in the longitudinal direction. In this case, the front, rear, left, right, up and down are defined with the side on which one spout 11b between two spouts 11b is formed as "front," and the side on which the other spout 11b is formed as "rear."

The vessel body 11 can be formed of, for example, a resin by injection molding, vacuum molding, or pressure molding. In addition, the vessel body 11 can be formed of, for example, a transparent or translucent resin. When the vessel body 11 is made transparent or translucent, the membrane filter 100 and the culture solution inside can be confirmed.

As shown in FIG. 3, the vessel body 11 has a pair of side wall parts 20 adjacent to each other in a direction in which the opening 11a is shortest (left to right direction). As shown in FIG. 2, the lower parts of the pair of side wall parts 20 are curved in an arc shape. In the present embodiment, since the membrane filter 100 is circular, the lower parts of the pair of side wall parts 20 are curved in a semicircular shape.

The upper parts of the pair of side wall parts 20 are formed in a rectangular shape that is continuous with the arc shape of the lower parts, and are formed to be longer in the vertical direction than the vertical dimension of the lower parts. In the present embodiment, the vertical dimension of the upper parts of the pair of side wall parts 20 is equal to or larger than the radius of the membrane filter 100. That is, the upper parts of the pair of side wall parts 20 extend to a position higher than the upper end of the membrane filter 100 when vertically placed.

A liquid volume scale 12 is provided on the outer surface of the pair of side wall parts 20. At least one liquid volume scale 12 may be provided at a position higher than the upper end of the membrane filter 100 when vertically placed. When the culture solution is injected up to the liquid volume scale 12, the membrane filter 100 can be completely submerged in the liquid. The liquid volume scale 12 may be formed by printing on the vessel body 11, or may be formed by providing an unevenness on the outer surface of the vessel body 11.

As shown in FIG. 3, the distance between the facing surfaces of the pair of side wall parts 20 gradually increases from a bottom part 11c of the vessel body 11 toward the opening 11a. The pair of side wall parts 20 have a first facing surface 21 and a second facing surface 22. The first facing surface 21 extends from the bottom part 11c of the vessel body 11 to above the membrane filter 100. The first facing surface 21 forms a narrow space that can vertically accommodate the membrane filter 100.

The second facing surface 22 is connected to the upper end of the first facing surface 21, has a gentler inclination with respect to the horizontal plane than the inclination of the first facing surface 21, and extends to both sides in the left to right direction. The second facing surface 22 widens the width of the opening 11a in the direction in which it is shortest above the membrane filter 100, and makes it easier to insert the membrane filter 100 into the vessel body 11.

As shown in FIG. 2, in the peripheral parts of the pair of side wall parts 20, and parts other than the opening 11a including the spout 11b are connected by a front wall part 30 and a rear wall part 40. As shown in FIG. 4, the front wall part 30 has a curved surface 31 and an inclined surface 32. The inclined surface 32 is inclined downward from the lower end of the spout 11b. The inclined surface 32 faces the rear wall part 40 in the front to rear direction (the longitudinal direction of the opening 11a). The curved surface 31 extends from the lower end of the inclined surface 32 to the lowest end of the vessel body 11. The curved surface 31 forms the front part of the arc-shaped bottom part 11c of the vessel body 11 (refer to FIG. 2).

As shown in FIG. 4, the rear wall part 40 has a curved surface 41 and an inclined surface 42. The inclined surface 42 is inclined downward from the upper end of the rear wall part 40. The inclined surface 42 faces the inclined surface 32 of the front wall part 30 in the front to rear direction (the longitudinal direction of the opening 11a). The curved surface 41 extends from the lower end of the inclined surface 42 to the lowest end of the vessel body 11. The curved surface 41 faces the curved surface 31 of the front wall part 30 in the front to rear direction (the longitudinal direction of the opening 11a) and forms the rear part of the arc-shaped bottom part 11c of the vessel body 11 (refer to FIG. 2).

In this manner, the culture vessel 10 according to the present embodiment has the opening 11a that opens in a slit shape, and the vessel body 11 that vertically accommodates the membrane filter 100 (sheet-like object) through the opening 11a. With such a configuration, as shown in FIG. 3, the accommodation space can be made deeper without increasing the internal volume of the vessel body 11, and thus the culture solution is less likely to spill, and the operation becomes easier. In addition, when the vessel body 11 vertically accommodates the membrane filter 100, the membrane filter 100 is less likely to float on the liquid surface. Therefore, it is possible to secure submersion of the membrane filter 100 in the liquid with a small amount of the liquid. Therefore, culture can be efficiently performed in a liquid medium using the membrane filter 100.

In addition, in the present embodiment, the vessel body 11 has the pair of side wall parts 20 adjacent to each other in a direction in which the opening 11a is shortest, and the lower parts of the pair of side wall parts 20 are curved in an arc shape as shown in FIG. 2. With such a configuration, the dead space in the vessel body 11 is reduced, and the membrane filter 100 can be submerged in the liquid even with a small amount of the liquid.

In addition, in the present embodiment, the upper parts of the pair of side wall parts 20 are formed in a rectangular shape that is continuous with the arc shape of the lower parts, and are formed to be longer in the vertical direction than the vertical dimension of the lower parts. With such a configuration, the membrane filter 100 can be inserted from the opening 11a of the vessel body 11 to the bottom part 11c without being bent or deformed.

In addition, in the present embodiment, as shown in FIG. 3, the distance between facing surfaces of the pair of side wall parts 20 increases from the bottom part 11c of the vessel body 11 toward the opening 11a. With such a configuration, it becomes easier to accommodate the membrane filter 100 in the vessel body 11. In addition, when the vessel body 11 is formed by injection molding, vacuum molding, pressure molding or the like, it is easily released from a mold, and the shape is easily formed.

In addition, in the present embodiment, as shown in FIG. 2 and FIG. 4, the inclined spout 11b is formed in at least a part of the opening 11a. With such a configuration, the culture solution after microorganisms are cultured can be easily transferred to another vessel, and the process can easily proceed to the next process.

As described above, according to the present embodiment, it is possible to provide the culture vessel 10 which is less likely to spill a liquid, is easy to operate, and can secure submersion of a sheet-like object in a liquid in the vessel body 11, and the microorganism culture system and nucleic acid analysis system using the culture vessel 10.

### [Second embodiment]

Next, a second embodiment of the present invention will be described. In the following description, the same or equivalent components as those in the above embodiment will be denoted with the same reference numerals, and the description thereof will be simplified or omitted.

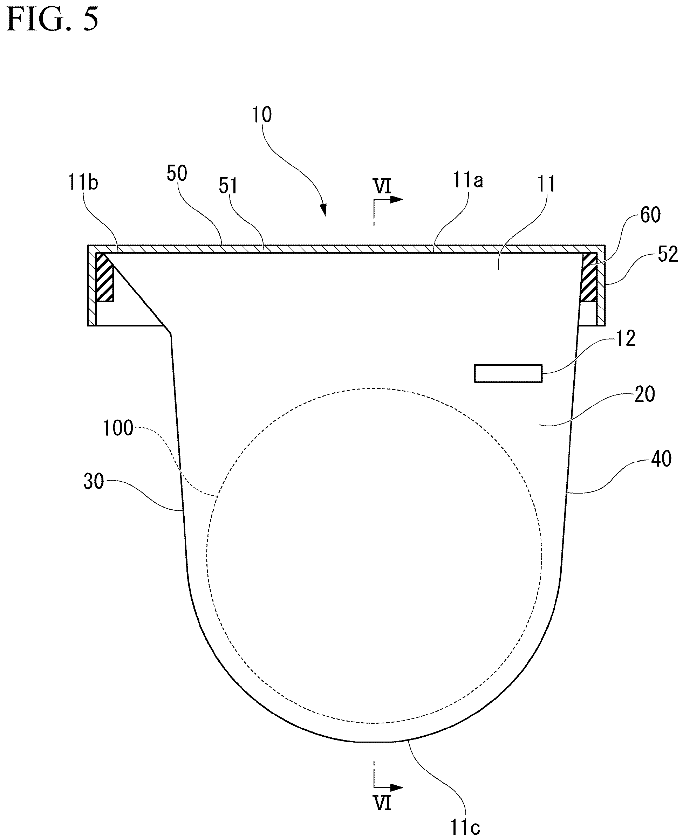

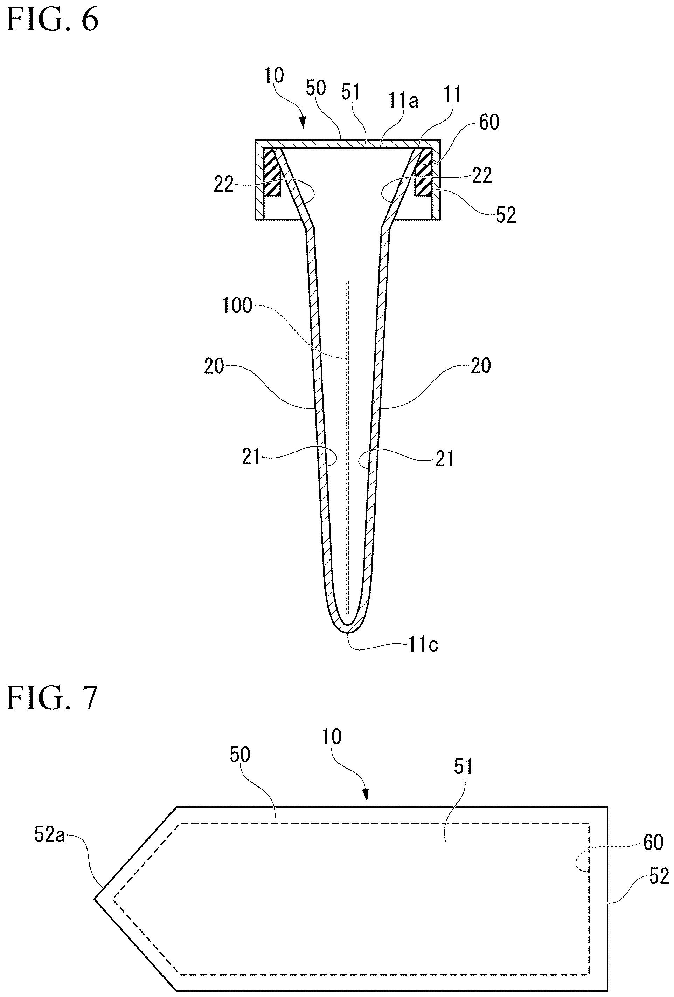

FIG. 5 is a partial cut-away side view of the culture vessel 10 according to the second embodiment. FIG. 6 is a cross-sectional view taken along the arrow VI-VI shown in FIG. 5. FIG. 7 is a plan view of the culture vessel 10 according to the second embodiment.

As shown in these drawings, the culture vessel 10 according to the second embodiment has a lid part 50 that covers at least a part of the opening 11a.

The lid part 50 is formed in a cylindrical shape with a top. The lid part 50 has a top wall part 51 and a peripheral wall part 52. The top wall part 51 covers the entire opening 11a including the spout 11b of the vessel body 11. The top wall part 51 has a right-angled pentagonal shape with a convex front side (the side of the spout 11b) in a plan view shown in FIG. 7.

As shown in FIG. 6, the peripheral wall part 52 is vertically provided downward from the peripheral part of the top wall part 51, and surrounds the opening 11a of the vessel body 11 from the side. A sealing part 60 may be additionally provided inside the peripheral wall part 52. The sealing part 60 seals the gap between the lid part 50 and the vessel body 11. The sealing part 60 can be formed of, for example, a flexible rubber, an elastomer or the like. Alternatively, the vessel body 11 and the lid part 50 may be fitted together to form a sealing structure.

In this manner, the culture vessel 10 according to the second embodiment has the lid part 50 that covers at least a part of the opening 11a. With such a configuration, for example, when shaking culture is performed, the lid part 50 can prevent the culture solution in the vessel body 11 from leaking to the outside. In addition, when the opening 11a is closed, it is possible to prevent contamination caused by microorganisms, foreign substances and the like floating in the air falling into the vessel body 11.

In addition, in the present embodiment, the sealing part 60 that seals the gap between the lid part 50 and the vessel body 11 is provided. With such a configuration, it is possible to reliably prevent leakage of the culture solution and contamination by floating substances. Here, in the case of static culture, the sealing part 60 is not necessary because there is less possibility of the culture solution leaking.

### [Third embodiment]

Next, a third embodiment of the present invention will be described. In the following description, the same or equivalent components as those in the above embodiment will be denoted with the same reference numerals, and the description thereof will be simplified or omitted.

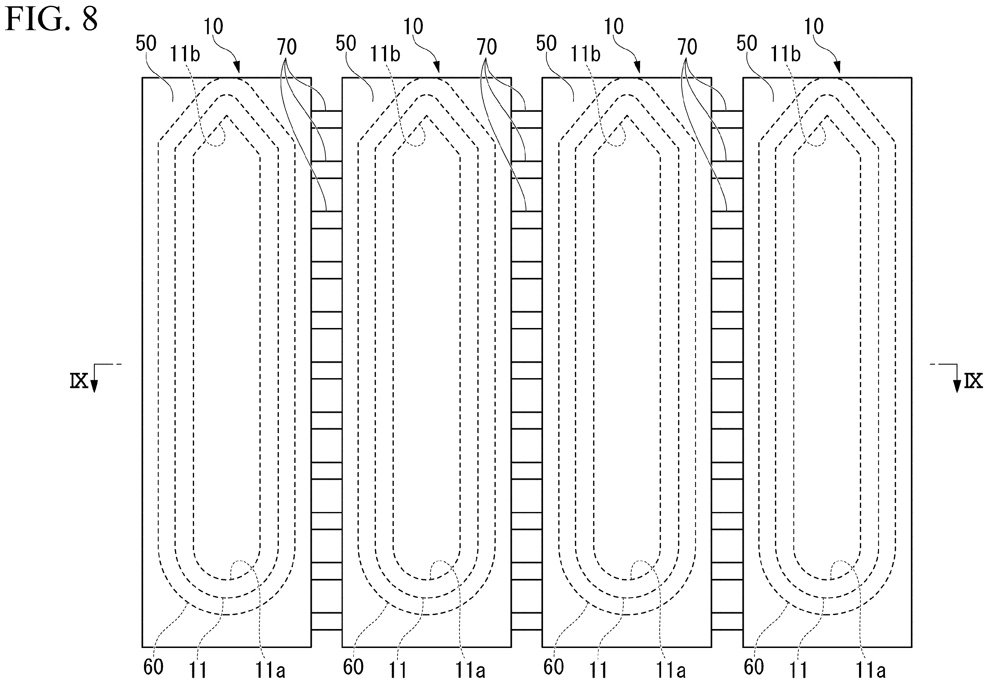

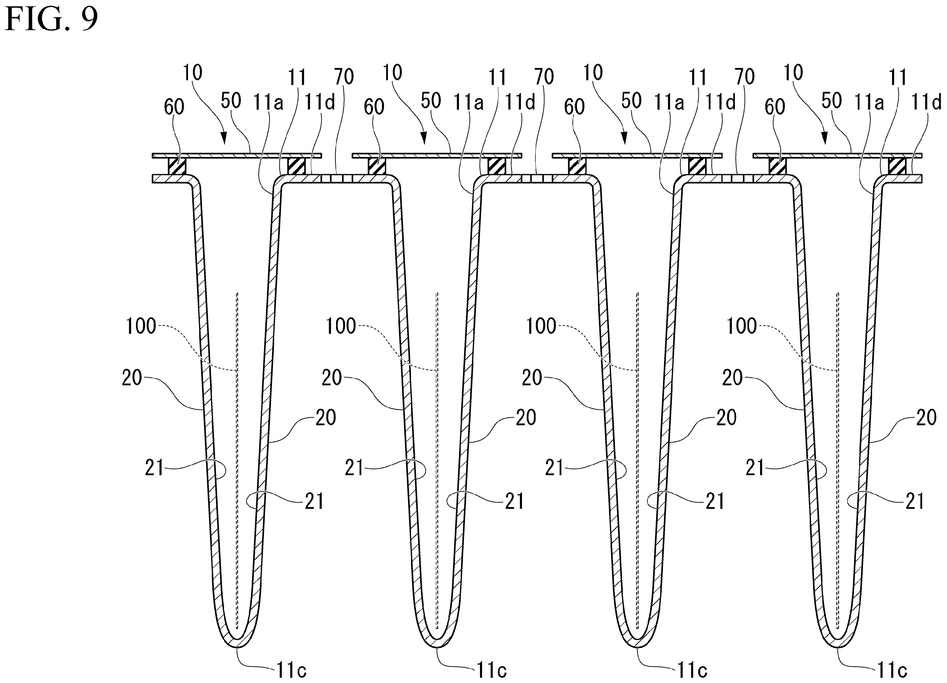

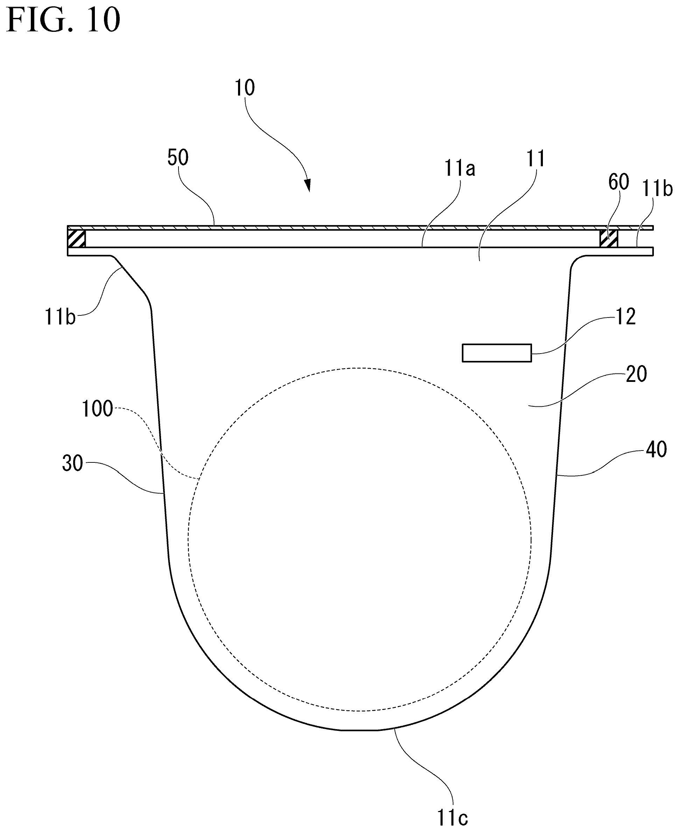

FIG. 8 is a plan view of the culture vessel 10 according to the third embodiment. FIG. 9 is a cross-sectional view taken along the arrow IX-IX shown in FIG. 8. FIG. 10 is a partial cut-away side view of the culture vessel 10 according to the third embodiment.

As shown in these drawings, in the culture vessel 10 according to the third embodiment, a plurality of vessel bodies 11 are connected to each other via connecting parts 70.

As shown in FIG. 8, the connecting part 70 connects the vessel bodies 11 to each other in the left to right direction (short direction). In the third embodiment, as shown in FIG. 9, a flange part 11d is formed on the opening edge of the opening 11a of the vessel body 11, and the connecting part 70 connects the vessel bodies 11 to each other via the flange part 11d. Here, the connecting part 70 may be directly connected from one of the pair of side wall parts 20 to the side wall part 20 of the other vessel body 11. Such a connecting part 70 can be integrally molded with the vessel body 11.

The connecting part 70 may have a breakable weakened part. As shown in FIG. 8, the weakened part of the present embodiment is formed of a plurality of thin columnar parts that connect the vessel bodies 11 together. This allows the connecting parts 70 to be broken and separation into individual vessel bodies 11 can be performed. Here, as the weakened part, for example, when the flange parts 11d are connected together, perforated holes may be formed in the flange parts 11d to make them have weaker rigidity than other parts, or a part of the flange part 11d may be desirably weakened by forming a straight or curved part that has a smaller width in the left to right direction or a smaller thickness in the vertical direction than other flange parts.

The lid part 50 according to the third embodiment is formed of, for example, a flexible film. The film (the lid part 50) can be attached to and detached from the flange part 11d of the vessel body 11 by the sealing part 60 formed of an adhesive. Here, the lid part 50 may have air permeability. For example, when the lid part 50 is formed of an oxygen-permeable film, it is possible to promote culture of microorganisms when the microorganisms to be cultured are aerobic microorganisms.

In this manner, in the culture vessel 10 according to the third embodiment, a plurality of vessel bodies 11 are connected to each other via the connecting parts 70. With such a configuration, in the microorganism test, even when a plurality of tests are performed simultaneously, the plurality of tests can be performed in a centralized manner, and the management and operation of the culture vessel 10 are not complicated.

In addition, in the third embodiment, the connecting part 70 has a breakable weakened part. With such a configuration, the connected vessel bodies 11 can be individually separated for culturing and testing according to the purpose, and thus the operability is improved.

In addition, in the third embodiment, the lid part 50 is a film, and the sealing part 60 is an adhesive that allows the film to be attached and detached. With such a configuration, since the lid part 50 is a film, it is possible to easily open and close the opening 11a of the vessel body 11. In addition, the lid part 50 can be easily positioned while avoiding interference between the lid part 50 and the connecting part 70.

In addition, in the third embodiment, the lid part 50 has air permeability. Here, in the second embodiment and a fifth embodiment to be described below, the lid part 50 may have air permeability. For example, when the lid part 50 is formed of an oxygen-permeable film, it is possible to promote culture of microorganisms when the microorganisms to be cultured are aerobic microorganisms.

Here, the lid part 50 may not have air permeability. For example, when the microorganisms to be cultured are anaerobic microorganisms, it is possible to prevent the inflow of oxygen and the like from the outside of the culture vessel 10, and it is possible to promote culture of anaerobic microorganisms.

### [Fourth embodiment]

Next, a fourth embodiment of the present invention will be described. In the following description, the same or equivalent components as those in the above embodiment will be denoted with the same reference numerals, and the description thereof will be simplified or omitted.

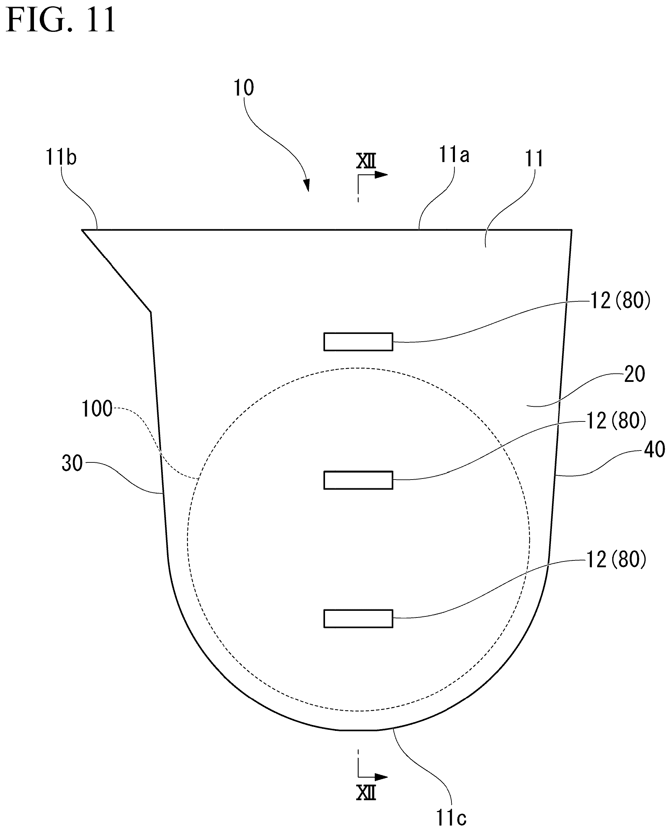

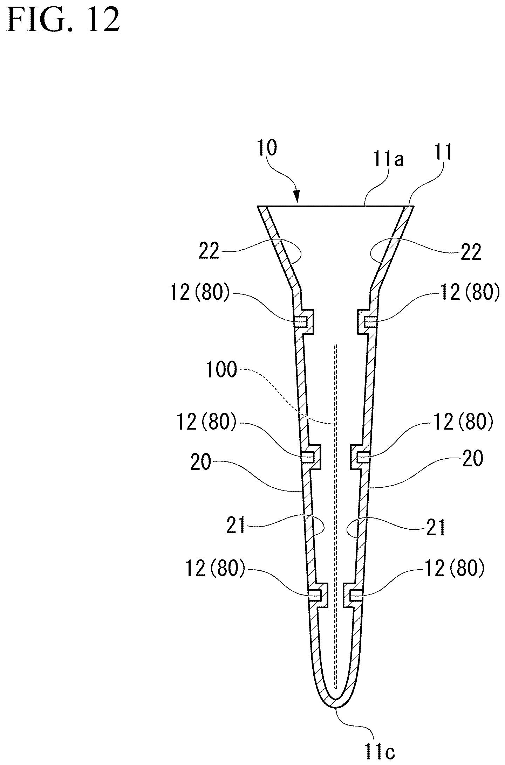

FIG. 11 is a side view of the culture vessel 10 according to the fourth embodiment. FIG. 12 is a cross-sectional view taken along the arrow XII-XII shown in FIG. 11.

As shown in these drawings, in the culture vessel 10 according to the fourth embodiment, protruding parts 80 that protrude toward the inside of the vessel body 11 are formed in the pair of side wall parts 20.

As shown in FIG. 11, the protruding parts 80 are concave parts formed on the outer surface of the pair of side wall parts 20. The plurality of protruding parts 80 are formed at intervals in the vertical direction, and form the above liquid volume scale 12. The protruding parts 80 are arranged in a row in the vertical direction so that they pass through the center position of the membrane filter 100.

As shown in FIG. 12, the protruding parts 80 are convex parts in the vessel body 11 and partially reduce the distance between the first facing surfaces 21 of the pair of side wall parts 20. Here, the position of the protruding part 80 provided in one of the pair of side wall parts 20 in the vertical direction matches the position of the protruding part 80 provided in the other side wall part, but the positions in the vertical direction may be different from each other.

In this manner, in the culture vessel 10 according to the fourth embodiment, the protruding parts 80 that protrude toward the inside of the vessel body 11 are formed in the pair of side wall parts 20. With such a configuration, the protruding part 80 prevents the membrane filter 100 from adhering to the first facing surfaces 21 of the pair of side wall parts 20, and makes it easier to supply the culture solution to microorganisms supported on the surface of the membrane filter 100.

### [Fifth embodiment]

Next, a fifth embodiment of the present invention will be described. In the following description, the same or equivalent components as those in the above embodiment will be denoted with the same reference numerals, and the description thereof will be simplified or omitted.

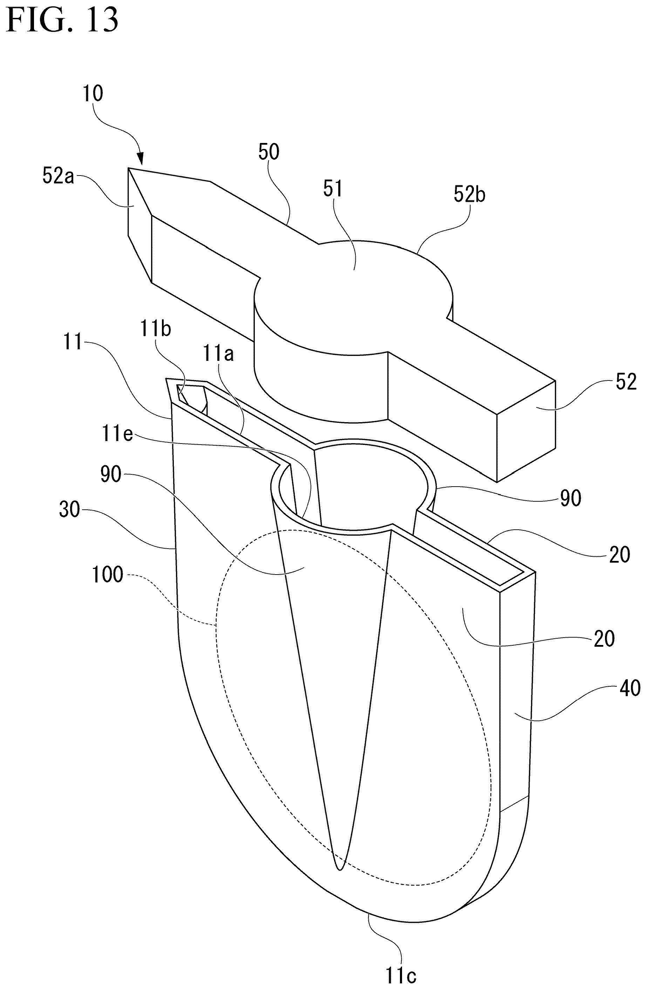

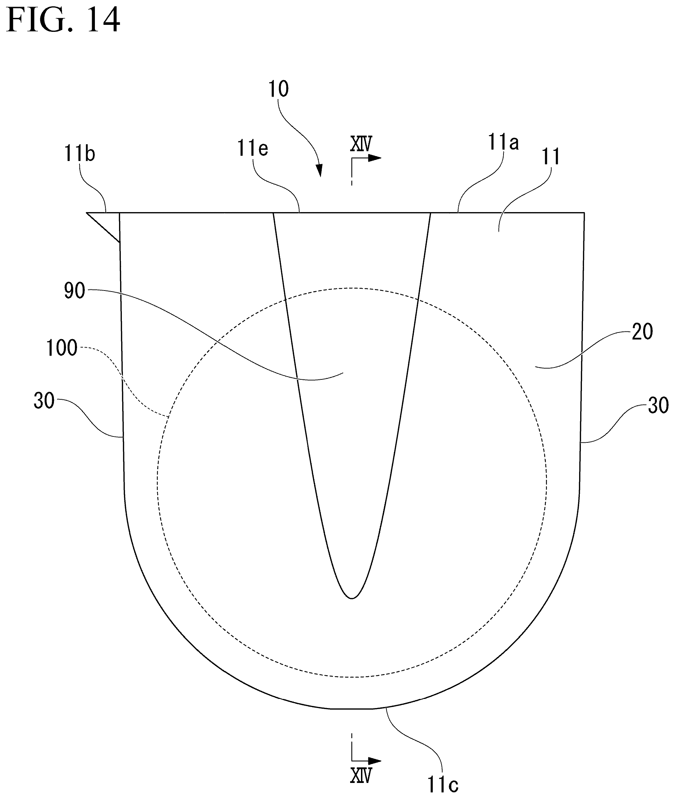

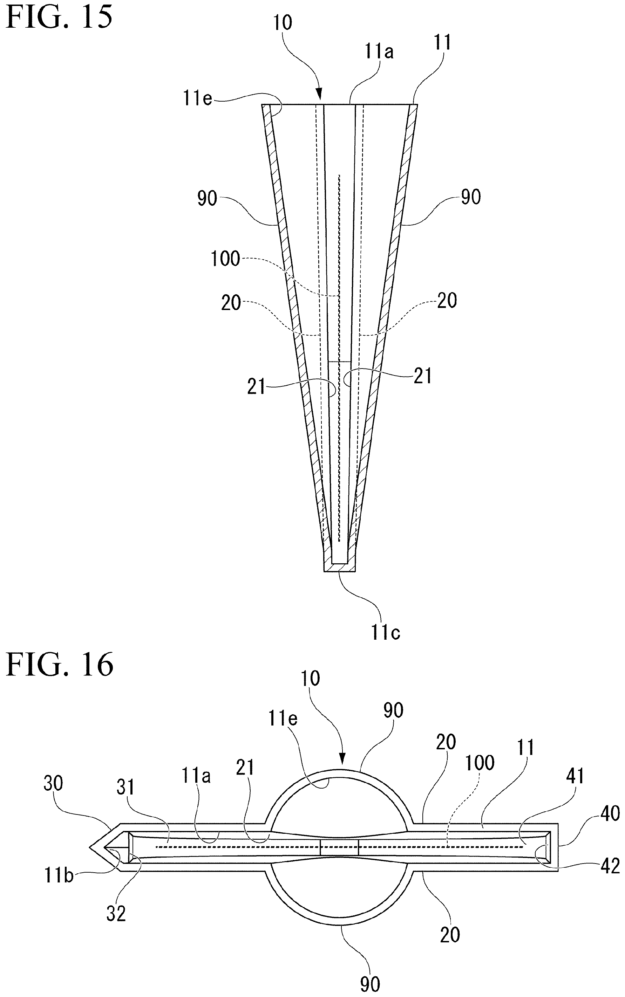

FIG. 13 is an exploded perspective view of the culture vessel 10 according to the fifth embodiment. FIG. 14 is a side view of the vessel body 11 according to the fifth embodiment. FIG. 15 is a cross-sectional view taken along the arrow XV-XV shown in FIG. 14. FIG. 16 is a plan view of the vessel body 11 according to the fifth embodiment.

As shown in these drawings, in the culture vessel 10 according to the fifth embodiment, inverted cone-shaped bulging parts 90 that partially widen the opening 11a in the direction in which it is shortest are formed in the pair of side wall parts 20.

The bulging parts 90 are convex parts formed on the outer surface of the pair of side wall parts 20. The bulging parts 90 are formed from the upper ends of the pair of side wall parts 20 toward the lowest end of the vessel body 11. The bulging part 90 becomes a concave part in the vessel body 11, and forms an enlarged diameter part 11e that partially widens the central part of the opening 11a in the longitudinal direction in the direction in which it is shortest as shown in FIG. 16.

The enlarged diameter part 11e has a circular shape in a plan view, and as shown in FIG. 15 and FIG. 16, the opening area thereof decreases toward the lowest end of the vessel body 11. Here, as shown in FIG. 13, in the lid part 50, a cylindrical accommodation tube 52b that accommodates the bulging part 90 is formed.

In this manner, in the culture vessel 10 according to the fifth embodiment, in the pair of side wall parts 20, the inverted cone-shaped bulging parts 90 that partially widen the opening 11a in the direction in which it is shortest are formed. With such a configuration, even if the distance between the first facing surfaces 21 of the pair of side wall parts 20 is small, a pipette, a syringe or the like can be inserted into the vessel body 11 through the enlarged diameter part 11e formed by the bulging part 90. When microorganisms leave the membrane filter 100 after culture, and float in the culture solution, they may precipitate due to gravity, and accumulate thickly in the lower part of the vessel body 11. In this case also, the tip of the pipette or syringe can be inserted to the lower part of the vessel body 11, and the culture solution in the lower part of the vessel body 11 can be collected. In addition, the enlarged diameter part 11e allows the pipette or syringe to collect the culture solution from any height position of the vessel body 11.

While preferable embodiments of the present invention have been described above with reference to the drawings, the present invention is not limited to the embodiments. The shapes, combinations and the like of the components shown in the above embodiments are only examples, and various modifications can be made based on design requirements and the like without departing from the spirit of the present invention.

For example, the vessel body 11 and the lid part 50 may be made of a material having appropriate flexibility, uneven parts may be formed so that the vessel body 11 and the lid part 50 fit together, and sealing may be performed by fitting the unevennesses. With such a configuration, there is no need to use materials such as adhesives that are different from the vessel body 11 and the lid part 50, and it is possible to prevent eluted chemical substances from foreign substances such as adhesives from flowing into the culture solution.

In addition, for example, in the above embodiment, the membrane filter 100 has been exemplified as the sheet-like object, but the object is not limited thereto, and a paper object, a plate object, a strip object, a film object, a sheet object, and other sheet-like objects may be used as long as they can support and culture microorganisms.

### REFERENCE SIGNS LIST

1 Nucleic acid analysis system
2 Microorganism collection system
3 Microorganism culture system
4 Nucleic acid extraction system
5 Hybridization reaction system
6 Detection system
10 Culture vessel
11 Vessel body
11a Opening
11b Spout
11c Bottom part
11d Flange part
11e Enlarged diameter part
12 Liquid volume scale
20 Side wall part
21 First facing surface
22 Second facing surface
30 Front wall part
31 Curved surface
32 Inclined surface
40 Rear wall part
41 Curved surface
42 Inclined surface
50 Lid part
51 Top wall part
52 Peripheral wall part
52b Accommodation tube
60 Sealing part
70 Connecting part
80 Protruding part
90 Bulging part
100 Membrane filter
200 Sample

## Claims

1. A culture vessel comprising an opening that opens in a slit shape, and a vessel body that vertically accommodates a sheet-like object through the opening.

2. The culture vessel according to claim 1,
wherein the vessel body has a pair of side wall parts adjacent to each other in a direction in which the opening is shortest, and
lower parts of the pair of side wall parts are curved in an arc shape.

3. The culture vessel according to claim 2,
wherein upper parts of the pair of side wall parts are formed in a rectangular shape that is continuous with the arc shape, and are formed longer in a vertical direction than a vertical dimension of the lower parts.

4. The culture vessel according to claim 2,
wherein the distance between facing surfaces of the pair of side wall parts increases from a bottom part of the vessel body toward the opening.

5. The culture vessel according to claim 1,
wherein an inclined spout is formed in at least a part of the opening.

6. The culture vessel according to claim 1, comprising a lid part that covers at least a part of the opening.

7. The culture vessel according to claim 6,
wherein the lid part has air permeability.

8. The culture vessel according to claim 6, comprising a sealing part that seals a gap between the lid part and the vessel body.

9. The culture vessel according to claim 8,
wherein the lid part is a film, and
the sealing part is an adhesive that allows the film to be attached and detached.

10. The culture vessel according to claim 1,
wherein a plurality of vessel bodies are connected to each other via connecting parts.

11. The culture vessel according to claim 10,
wherein the connecting part has a breakable weakened part.

12. The culture vessel according to claim 2,
wherein, in the pair of side wall parts, inverted cone-shaped bulging parts that partially widen the opening in the direction in which it is shortest are formed.

13. The culture vessel according to claim 2,
wherein, in the pair of side wall parts, protruding parts that protrude toward the inside of the vessel body are formed.

14. A microorganism culture system that cultures microorganisms using the culture vessel according to any one of claims 1 to 13.

15. A nucleic acid analysis system comprising the microorganism culture system according to claim 14, and configured to analyze nucleic acids extracted from the microorganisms.
